# EUROPEAN PATENT SPECIFICATION

(11) **EP 3 740 257 B1**
(45) Date of publication and mention of the grant of the patent: **12.07.2023**
(21) Application number: 19703501.7
(22) Date of filing: 15.01.2019
(51) Int. Cl.: A61M 1/00, A61B 5/00

(54) **WOUND SENSOR AND DIAGNOSTICS SYSTEM FOR WOUND THERAPY APPLICATIONS**
WUNDSENSOR UND DIAGNOSESYSTEM FÜR WUNDTHERAPIEANWENDUNGEN
CAPTEUR DE PLAIES ET SYSTÈME DE DIAGNOSTIC POUR DES APPLICATIONS DE TRAITEMENT DE PLAIES

(30) Priority: 15.01.2018 US 201862617487 P
(43) Date of publication of application: 25.11.2020
(73) Proprietor: 3M Innovative Properties Company, St. Paul, MN 55133-3427 (US)
(72) Inventor: LOCKE, Christopher, Brian, Bournemouth BH9 3SD (GB); COULTHARD, Richard Daniel, John, Verwood BH31 6LL (GB); LONG, Justin, Alexander, Bournemouth BH1 1PP (GB)
(74) Representative: Simmons & Simmons
(86) International application number: PCT/US2019/013634
(87) International publication number: WO 2019/140441

(56) References cited:
- EP-A1- 2 331 162
- WO-A1-2015/073885
- US-A1- 2011 015 587
- US-A1- 2014 005 618

## Description

### TECHNICAL FIELD

The invention set forth in the appended claims relates generally to tissue treatment systems and more particularly, but without limitation, to sensors as well as diagnostic and control systems for wound therapy applications.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound can be washed out with a stream of liquid solution, or a cavity can be washed out using a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and instillation therapy are widely known, improvements to therapy systems, components, and processes may benefit healthcare providers and patients.

EP 2 331 162 discloses a negative pressure treatment system with a MEMs sensor to monitor wound parameters.

US2014005618A1 discloses a negative pressure treatment system utilizing an RFID sensor in a dressing to measure pressure data.

WO2015/073885 discloses a system for providing controlled electrolysis treatment to a site in tissue.

### BRIEF SUMMARY

The invention is defined by the claims in which there is required a system for treating a tissue site, comprising: a dressing adapted to be placed on the tissue site; a diagnostic module adapted to be positioned adjacent the dressing and comprising: a first sensor configured to detect a pH level of fluid present at the tissue site and to generate a first output based on the detected pH level, the first sensor comprising a first transceiver configured to transmit the first output; a first remote sensor device comprising a second sensor, wherein the second sensor is configured to detect a secondary pH level and to generate a second output based on the detected secondary pH level, the first remote sensor device comprising a second transceiver configured to transmit the second output generated by the second sensor; a negative-pressure source adapted to be fluidly coupled to the dressing; a first fluid source adapted to be fluidly coupled to the dressing and to deliver a first fluid to the dressing; and a controller configured to receive the first output and control the negative-pressure source and the fluid source based on the first output from the first sensor and to receive the second output and compare the first output to the second output.

A selection of optional features is set out in the dependent claims.

Objectives, advantages, and a preferred mode of making and using the claimed subject matter may be understood best by reference to the accompanying drawings in conjunction with the following detailed description of illustrative embodiments.

Insofar as the term invention or embodiment is used in the following, or features are presented as being optional, this should be interpreted in such a way that the only protection sought is that of the invention claimed.

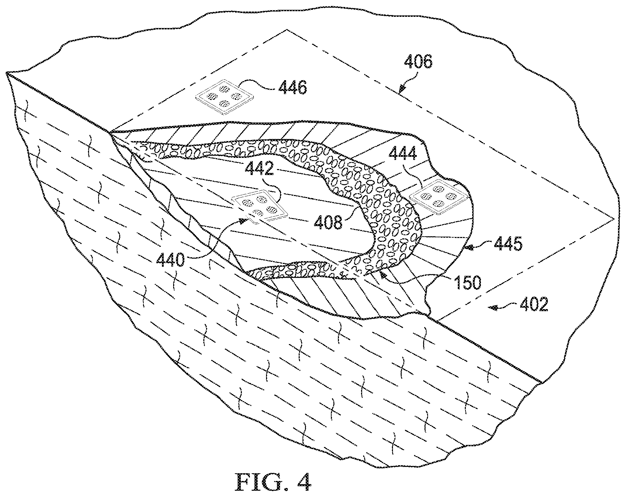

Reference(s) to "embodiment(s)" throughout the description which are not under the scope of the appended claims merely represent possible exemplary executions and are not part of the present invention. Embodiments falling under the claimed invention are described in figures 4 to 6.

### BRIEF DESCRIPTION OF THE DRAWINGS

Figure 1 is a functional block diagram of an example embodiment of a therapy system that can provide negative-pressure therapy to a tissue site in accordance with this specification;
Figure 2 is a schematic diagram illustrating additional details that may be associated with an example embodiment of the therapy system of Figure 1;
Figure 3 is an exploded view of a dressing, according to some illustrative embodiments, suitable for use with the therapy system of Figure 1, depicted without a dressing interface and with an illustrative embodiment of a release liner for protecting the dressing prior to application at a tissue site;
Figure 4 is a schematic view of an illustrative embodiment of a portion of the therapy system of Figure 1, illustrating additional features according to some embodiments;
Figure 5 is a schematic diagram illustrating additional details of a diagnostic module that may be associated with some embodiments of the therapy system of Figure 1;
Figure 6 is a schematic diagram illustrating additional details of a diagnostic module that may be associated with some additional embodiments of the therapy system of Figure 1;
Figure 7 is a schematic view of an illustrative embodiment of a portion of the therapy system of Figure 1, illustrating additional details that may be associated with some embodiments;
Figure 8 is a schematic view of an illustrative embodiment of a portion of the therapy system of Figure 1, illustrating additional features according to some embodiments; and
Figure 9 is a flow chart illustrating a method of operation of the therapy system of Figure 1, according to some example embodiments.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

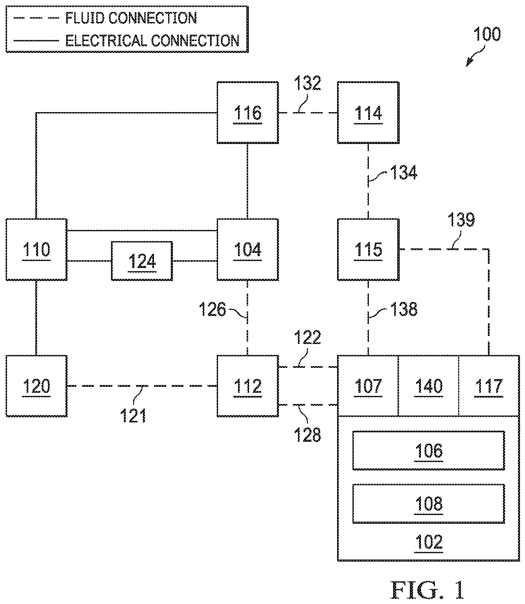

Figure 1 is a simplified functional block diagram of an example embodiment of a therapy system 100 that can provide negative-pressure therapy with instillation of topical treatment solutions in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a negative-pressure supply, and may include or be configured to be coupled to a distribution component, such as a dressing. In general, a distribution component may refer to any complementary or ancillary component configured to be fluidly coupled to a negative-pressure supply in a fluid path between a negative-pressure supply and a tissue site. A distribution component is preferably detachable, and may be disposable, reusable, or recyclable. For example, a dressing 102 may be fluidly coupled to a negative-pressure source 104, as illustrated in Figure 1. A dressing may include a cover, a tissue interface, or both in some embodiments. The dressing 102, for example, may include a cover 106 and a tissue interface 108. A regulator or a controller, such as a controller 110, may also be coupled to the negative-pressure source 104.

In some embodiments, a dressing interface, such as dressing interface 107, may facilitate coupling the negative-pressure source 104 to the dressing 102. For example, such a dressing interface may be a T.R.A.C.^{®} Pad or Sensa T.R.A.C^{®} Pad available from KCI of San Antonio, Texas. In some embodiments, the dressing 102 may include a dressing interface 107 as well as a second dressing interface 117. The therapy system 100 may optionally include a fluid container, such as a container 112, coupled to the dressing 102 and to the negative-pressure source 104.

The therapy system 100 may also include a source of instillation solution. For example, a solution source 114 may be fluidly coupled to the dressing 102, as illustrated in the example embodiment of Figure 1. The solution source 114 may be fluidly coupled to a positive-pressure source, such as the positive-pressure source 116, in some embodiments, or may be fluidly coupled to the negative-pressure source 104. A regulator, such as an instillation regulator 115, may also be fluidly coupled to the solution source 114 and the dressing 102. In some embodiments, the instillation regulator 115 may also be fluidly coupled to the negative-pressure source 104 through the dressing 102, as illustrated in the example of Figure 1.

Additionally, the therapy system 100 may include sensors to measure operating parameters and provide feedback signals to a controller 110 indicative of the operating parameters. As illustrated in Figure 1, for example, the therapy system 100 may include a first sensor 120 and a second sensor 124, or both, coupled to the controller 110. The first sensor 120 may also be coupled or configured to be coupled to a distribution component and to the negative-pressure source 104.

Components may be fluidly coupled to each other to provide a path for transferring fluids (i.e., liquid and/or gas) between the components. For example, components may be fluidly coupled through a fluid conductor, such as a tube. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors may be molded into or otherwise integrally combined with other components. Coupling may also include mechanical, thermal, electrical, or chemical coupling (such as a chemical bond) in some contexts. For example, one or more tubes, such as conduits 122 and 128, may mechanically and fluidly couple the dressing 102 to the container 112 in some embodiments.

In general, components of the therapy system 100 may be coupled directly or indirectly. For example, the negative-pressure source 104 may be directly coupled to the controller 110, and may be indirectly coupled to the dressing interface 107 through the container 112 by conduit 126 and conduit 128. The first sensor 120 may be fluidly coupled to the dressing 102 directly or indirectly by conduit 121 and conduit 122. Additionally, the positive pressure source 116 may be coupled indirectly to the dressing interface 107 through the solution source 114 and the instillation regulator 115 by fluid conductors 132, 134, and 138. Alternatively, the positive pressure source 116 may be coupled indirectly to the second dressing interface 117 through the solution source 114 and the instillation regulator 115 by fluid conductors 132, 134, and 139.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A negative-pressure supply, such as the negative-pressure source 104, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. For example, in some embodiments, the negative-pressure source 104 may be combined with the controller 110 and other components into a therapy unit. A negative-pressure supply may also have one or more supply ports configured to facilitate coupling and de-coupling the negative-pressure supply to one or more distribution components.

The tissue interface 108 can be generally adapted to contact a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the nature and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

In some embodiments, the tissue interface 108 may be a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, the pathways of a manifold may be interconnected to improve distribution or collection of fluids across a tissue site. In some illustrative embodiments, a manifold may be a porous foam material having interconnected cells or pores. For example, cellular foam, open-cell foam, reticulated foam, porous tissue collections, and other porous material such as gauze or felted mat generally include pores, edges, and/or walls adapted to form interconnected fluid channels. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

The average pore size of a foam may vary according to needs of a prescribed therapy. For example, in some embodiments, the tissue interface 108 may be a foam having pore sizes in a range of 400-600 microns. The tensile strength of the tissue interface 108 may also vary according to needs of a prescribed therapy. For example, the tensile strength of a foam may be increased for instillation of topical treatment solutions. In one non-limiting example, the tissue interface 108 may be an open-cell, reticulated polyurethane foam such as a GRANUFOAM^{™} dressing or a V.A.C. VERAFLO^{™} dressing, both available from Kinetic Concepts, Inc. of San Antonio, Texas.

The tissue interface 108 may be either hydrophobic or hydrophilic. In an example in which the tissue interface 108 may be hydrophilic, the tissue interface 108 may also wick fluid away from a tissue site, while continuing to distribute negative pressure to the tissue site. The wicking properties of the tissue interface 108 may draw fluid away from a tissue site by capillary flow or other wicking mechanisms. An example of a hydrophilic foam is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

The tissue interface 108 may further promote granulation at a tissue site when pressure within the sealed therapeutic environment is reduced. For example, any or all of the surfaces of the tissue interface 108 may have an uneven, coarse, or jagged profile that can induce microstrains and stresses at a tissue site if negative pressure is applied through the tissue interface 108.

In some embodiments, the tissue interface 108 may be constructed from bioresorbable materials. Suitable bioresorbable materials may include, without limitation, a polymeric blend of polylactic acid (PLA) and polyglycolic acid (PGA). The polymeric blend may also include without limitation polycarbonates, polyfumarates, and capralactones. The tissue interface 108 may further serve as a scaffold for new cell-growth, or a scaffold material may be used in conjunction with the tissue interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may be, for example, an elastomeric film or membrane that can provide a seal adequate to maintain a negative pressure at a tissue site for a given negative-pressure source. The cover 106 may have a high moisture-vapor transmission rate (MVTR) in some applications. For example, the MVTR may be at least 250 g/m^2 per twenty-four hours in some embodiments, measured using an upright cup technique according to ASTM E96/E96M Upright Cup Method at 38°C and 10% relative humidity (RH). In some embodiments, a MVTR up to 5,000 grams per square meter per twenty-four hours may provide may provide effective breathability and mechanical properties. In some example embodiments, the cover 106 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive that extends about a periphery, a portion, or an entire sealing member of the cover 106. In some embodiments, for example, some or all of the cover 106 may be coated with an acrylic adhesive having a coating weight between 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

A controller, such as the controller 110, may be a microprocessor or computer programmed to operate one or more components of the therapy system 100, such as the negative-pressure source 104. In some embodiments, for example, the controller 110 may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the tissue interface 108, for example. The controller 110 is also preferably configured to receive one or more input signals, such as a feedback signal, and programmed to modify one or more operating parameters based on the input signals.

Sensors, such as the first sensor 120 and the second sensor 124, are generally known in the art as any apparatus operable to detect or measure a physical phenomenon or property, and generally provide a signal indicative of the phenomenon or property that is detected or measured. For example, the first sensor 120 and the second sensor 124 may be configured to measure one or more operating parameters of the therapy system 100. In some embodiments, the first sensor 120 may be a transducer configured to measure pressure in a pneumatic pathway and convert the measurement to a signal indicative of the pressure measured. In some embodiments, for example, the first sensor 120 may be a piezoresistive strain gauge. The second sensor 124 may optionally measure operating parameters of the negative-pressure source 104, such as the voltage or current, in some embodiments. Preferably, the signals from the first sensor 120 and the second sensor 124 are suitable as an input signal to the controller 110, but some signal conditioning may be appropriate in some embodiments. For example, the signal may need to be filtered or amplified before it can be processed by the controller 110. Typically, the signal is an electrical signal, but may be represented in other forms, such as an optical signal.

The container 112 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

The solution source 114 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

The dressing 102 may further include an apparatus containing sensors for measuring one or more conditions or variables present at the dressing 102 and/or tissue site. For example, the dressing 102 may include diagnostic module 140, which may be positioned on, under, within, or between any of the other components of the dressing 102, for example the cover 106 and tissue interface 108. In some embodiments, the diagnostic module 140 may be positioned on or within either or both of the dressing interface 107 and/or second dressing interface 117.

In operation, the tissue interface 108 may be placed within, over, on, or otherwise proximate to a tissue site. The cover 106 may be placed over the tissue interface 108 and sealed to an attachment surface near the tissue site. For example, the cover 106 may be sealed to undamaged epidermis peripheral to a tissue site. Thus, the dressing 102 can provide a sealed therapeutic environment proximate to a tissue site, substantially isolated from the external environment, and the negative-pressure source 104 can reduce the pressure in the sealed therapeutic environment. Negative pressure applied across the tissue site through the tissue interface 108 in the sealed therapeutic environment can induce macrostrain and microstrain in the tissue site, as well as remove exudates and other fluids from the tissue site, which can be collected in container 112.

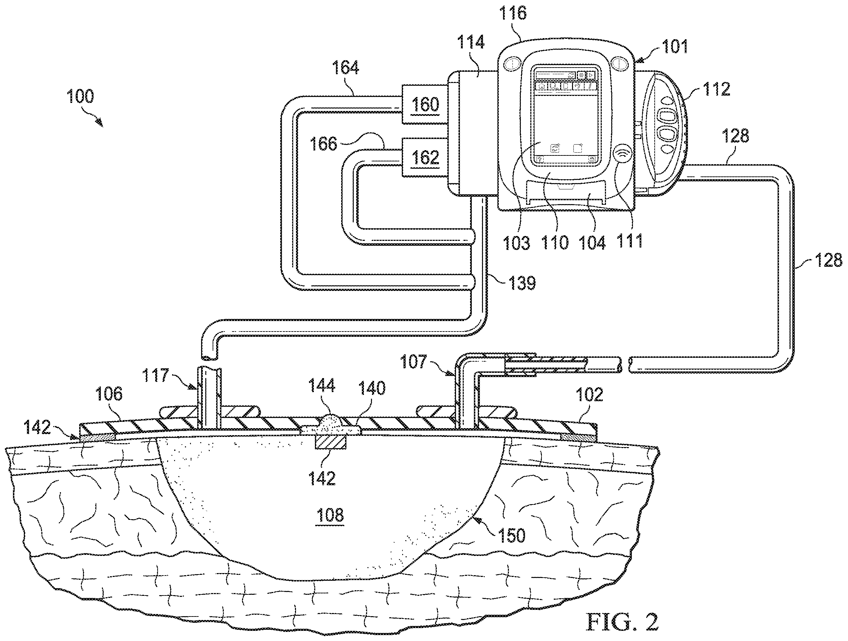

Figure 2 is a schematic diagram of an example embodiment of the therapy system 100, showing some further detail and additional features, including further detail with respect to a therapy unit 101 and diagnostic module 140. For example, one or more components of the therapy system 100 may be packaged as a single, integrated unit, such as therapy unit 101. For example, the therapy unit 101 may include the negative-pressure source 104, the controller 110, and positive pressure source 116. The therapy unit 101 may be, for example, a V.A.C.ULTA^{™} Therapy Unit available from Kinetic Concepts, Inc. of San Antonio, Texas. The therapy unit 101 may also include a display unit 103, which may be a graphical user interface (GUI) configured to both display data as well as receive input from a user. The display unit 103 may be configured to display data related to the diagnostic module 140. The display unit 103 may also be configured to display information related to the delivery of negative-pressure therapy and/or fluid instillation therapy to a tissue site 150. The therapy unit 101 may also include a communications device, which may be associated with the controller 110. For example, the therapy unit 101 may include communications device 111, which may be configured to exchange data with the diagnostic module 140. The communications device 111 may receive data from a communications module 144 of the diagnostic module 140 and communicate the data to the controller 110 of the therapy unit 101 for processing. The communications device 111 may be further configured to receive data and/or instructions from the controller 110 and transmit the data to the communications module 144 of the diagnostic module 140.

As shown in Figure 2, the diagnostic module 140 may be positioned within a portion of the dressing 102. For example, the diagnostic module 140 may be placed between the tissue interface 108 and the cover 106. However, in some embodiments, the diagnostic module 140 may be placed within the tissue interface 108, below the tissue interface 108 at the tissue site 150, within the cover 106, or within a portion of a dressing interface, such as dressing interface 107.

In some embodiments, the diagnostic module 140 may include a sensor device 142, which may be configured to measure one or more parameters in the environment of the tissue site 150. The one or more parameters measured by the sensor device 142 may be in addition to pressure measurements gathered by one or more pressure sensors, such as the first sensor 120, of the therapy system 100. For example, the sensor device 142 may be configured to detect and/or measure pH of wound exudates, temperature at the tissue site 150 as well as within the dressing 102, oxygen concentration of tissue at the tissue site 150 as well as in the surrounding tissue, humidity within the dressing 102, glucose levels within wound exudates, as well as other parameters. Thus, in some embodiments, the sensor device 142 may include one or more sensors, such as a pH sensor, temperature sensor, humidity sensor, blood sensor, glucose sensor, growth factor sensor, volatile organic compound (VOC) sensor, or another type of sensor. The sensor device 142 may also include a pressure sensor. Additionally, the sensor device 142 may include one or more sensors for detecting and/or measuring various electrolyte levels at a tissue site 150 through electrical resistance sensing.

As previously mentioned, the diagnostic module 140 may also include a communications component, such as communications module 144, for transmitting and receiving data to/from one or more other components of the therapy system 100, such as the therapy unit 101 or controller 110. For example, the communications module 144 may include a transceiver configured to exchange data with a communications device 111 that is part of the controller 110 or therapy unit 101 or electrically coupled to the controller 110. The communications module 144 may be configured to transmit data regarding the parameters detected and/or measured by the sensor device 142 of the diagnostic module 140. In some embodiments, the communications module 144 of the diagnostic module 140 may be configured to wirelessly exchange data with a communications device 111 that is electrically coupled to the controller 110. For example, the communications module 144 of the diagnostic module 140 and the communications device 111 of the controller 110 may be configured to communicate using the Bluetooth^{®} 4.0 protocol. Alternative wireless communication protocols may also be employed, including other Bluetooth^{®} standards, Zigbee^{®}, ANT, Z-WAVE, Wireless USB, or others. Other forms of wireless communication may also be incorporated, such as non-radio frequency technologies such as IrDA and Ultrasonic communications.

Alternatively or additionally, the diagnostic module 140 may be electrically coupled to the therapy unit 101, communications device 111, and/or the controller 110 via one or more wires, and thus the communications module 144 of the diagnostic module 140 may exchange data via a wired connection with the controller 110 related to one or more parameters sensed by the sensor device 142. For example, two or three wires may be implemented, which may provide electrical power to the diagnostic module 140 from the therapy unit 101, or more specifically the controller 110, as well as conduct multiplexed bidirectional signals.

In some embodiments, an optimized wired connection may be included to electrically couple the diagnostic module 140 to the therapy unit 101. In such embodiments, a number of wires may be used, which may in part depend on the number of individual sensors included as part of the sensor device 142 of the diagnostic module 140. Given that multiple individual sensors may be included as part of the sensor device 142, with each of the sensors potentially having different signal and communication requirements, a small interface micro-controller may be included as part of the diagnostic module 140 to reduce the total number of wires required for communication between the sensing device 142 of the diagnostic module 140 and the controller 110 of the therapy unit 101. For example, a micro-controller may be capable of polling the sensors of the diagnostic module 140 and digitizing the data from the sensors so that it can be communicated to the controller 110 of the therapy unit 101. In such embodiments, power for the diagnostic module 140 may be provided through the wired connection, while communications between the diagnostic module 140 and the controller 110 may use a Serial Perpheral Interface bus (SPI) or similar protocol to minimize the total number of wires required. Depending on the power requirements of the particular diagnostic module 140, a system such as a 1-Wire could be used. Incorporating a wired solution for enabling communications between the diagnostic module 140 and the therapy unit 101 may also increase the number and types of sensors that could be used in the sensor device 142 of the diagnostic module 140 by mitigating potential power limitations of using one or more batteries for powering the sensors.

Still referring primarily to Figure 2, the therapy system 100 may further include additional sources of solution or treatment compounds or substances, in addition to the solution source 114. For example, the therapy system 100 may include a first treatment source 160 and a second treatment source 162, both of which may be in fluid connection with the solution source 114. Each of the first treatment source 160 and the second treatment source 162 may include one or more compounds that may be delivered to the tissue site for therapeutic purposes. The first treatment source 160 and the second treatment source 162 may be arranged as part of the therapy system 100 so as to be able to modify a standard instillate solution provided by the solution source 114 by dosing with one or more additional compounds. As shown in Figure 2, the first treatment source 160 may be fluidly connected by first source conduit 164 to the fluid conduit 139 that connects the solution source 114 to dressing 102 via the second dressing interface 117. Similarly, the second treatment source 162 may also be fluidly connected by second source conduit 166 to fluid conduit 139. Thus, in operation, as instillation fluid is being conducted from the solution source 114 to the dressing 102 through fluid conduit 139, additional therapeutic compounds from either or both of the first treatment source 160 and the second treatment source 162 may be conducted through first source conduit 164 and second source conduit 166, respectively, and added to the instillation fluid. Additional treatment sources, such as a third treatment source and a fourth treatment source (not shown), may also be included in the therapy system 100, which may include additional treatment compounds for delivering to the tissue site.

In operation, in response to one or more parameters detected and/or sensed by the sensors of the sensor device 142, the diagnostic module 140 may transmit data from the sensor device 142 via the communications module 144 to the communications device 111 of the therapy unit 101. The controller 110 may receive data, via the communications device 111, from the diagnostic module 140 and process the data to determine one or more factors related to the tissue site 150. Based on one or more parameters measured by the diagnostic module 140, the controller 110 may be able to determine the status and healing trend of the tissue site 150. Based on an assessment of pH data, humidity data, temperature data, and/or data provided by the particular type(s) of sensor(s) included in the sensor device 142 of the diagnostic module 140, the controller 110 may determine a progression of wound healing. For example, a change in a pH measurement may signal a change in the status of the tissue site 150. In some instances, a tissue site, such as a wound, may be considered in a healthy state if the pH of the wound fluids stay within a particular range. Elevated or reduced pH measurements may indicate that a wound is in a chronic or inflammatory state. In some instances, the status of a tissue site 150 may be identified by monitoring increases in measured humidity and/or temperature. For example, if the temperature data indicates that the temperature at the tissue site 150 is increasing, the increasing temperature may be an indication that the wound is infected.

The controller 110 may then alert a user of the therapy system 100 as to the status of the tissue site 150, so that the user may take one or more actions to address or remedy the status of the tissue site 150. In some embodiments, the controller 110 may generate an output or alert to a user in order to direct the user to administer one or more forms of therapy. For example, the controller 110 may indicate via the display unit 103 of the therapy unit 101 that the user should increase fluid instillation therapy as well as administer a first therapeutic compound from the first treatment source 160. Following the administration of the first therapeutic compound and fluid instillation therapy, the therapy system 100 may continue to operate and measure the effects of the administered therapy via one or more parameters measured by the diagnostic module 140. Subsequently, the controller 110 may determine that, based on measurements taken by the diagnostic module 140, the status of the tissue site 150 has changed, and that an adjustment to one or more forms of therapy would be beneficial to the tissue site 150. For example, the controller 110 may generate an output to the user that delivery of the first therapeutic compound from the first treatment source 160 should be suspended, while fluid instillation therapy from the solution source 114 should be increased. The controller 110 may continue to monitor the status of the tissue site 150 through parameters measured by the diagnostic module 140 and indicate proposed therapy adjustments throughout the operation of the therapy system 100.

Furthermore, in some embodiments, the controller 110 may automatically direct that one or more types of therapy to the tissue site 150 could be initiated, adjusted, or stopped. In some embodiments, the controller 110 may automatically make changes to one or more forms of therapy, and thus the adjustments to the therapy may be triggered independently of an operator of the therapy system 100. However, the adjustments to the one or more forms of therapy may be within a set of bounds previously specified by the operator before activating the therapy system 100. In some embodiments, in response to a status of the tissue site 150 as determined by the controller 110, the controller 110 may adjust the application of negative pressure to the tissue site 150. For example, the negative-pressure source 104 may be directed by the controller 110 to reduce or cease delivery of negative pressure to the tissue site 150 for specific time periods, such as while fluid instillation therapy is being administered to the tissue site 150. Furthermore, the controller 110 may adjust the operation of the negative-pressure source 104 to maintain pressure levels within the dressing 102 at desired levels, based on feedback from a pressure sensor included as part of the diagnostic module 140 and/or a pressure sensor located at another portion of the therapy system 100, such as part of first sensor 120. Additionally or alternatively, in response to the status of the tissue site 150, the controller 110 may initiate the instillation of fluid, for example from solution source 114, to the tissue site 150. Further, the controller 110 may adjust one or more properties of the fluid administered to the tissue site 150. For example, in response to a particular status of the tissue site 150 as determined by the controller 110 in response to parameters sensed by the diagnostic module 140, the controller 110 may cause a treatment compound from the first treatment source 160 to be conducted through the first source conduit 164 and added to the instillation fluid being administered to the tissue site 150. Additionally or alternatively, the controller 110 may cause a treatment compound from the second treatment source 162 to be conducted through the second source conduit 166 and added to the instillation fluid being administered to the tissue site 150.

As conditions of the tissue site 150 change over time, the controller 110 may determine a new or changed status of the tissue site 150 based on data received from the diagnostic module 140. As a result, the controller 110 may adjust the therapy being administered to the tissue site 150. For example, in response to the changed status, the controller 150 may stop the delivery of one or both of the first treatment compound and the second treatment compound from the first treatment source 160 and the second treatment source 162, respectively. Furthermore, the controller 110 may stop the delivery of instillation fluid from the solution source 114 altogether, and/or cause the delivery of negative-pressure to the tissue site 150 to cease. Additionally, the controller 110 may provide feedback to a caregiver or the patient.

Furthermore, in some instances, the controller 110 may determine that it would be beneficial to alternate between two forms of fluid instillation therapy. For example, the controller 110 may direct the solution source 114 to direct a first fluid, such as a saline solution, to the tissue site 150 for promotion of granulation at the tissue site 150. The controller 110 may then suspend the operation of the negative-pressure source 104 for a determined period of time to allow the saline solution to remain in contact with the tissue site 150. The controller 110 may also take into account data received from the sensors of the diagnostic module 140 when determining the length of time for allowing the saline solution to remain at the tissue site 150. Following the appropriate period of time, the controller 110 may direct the negative-pressure source 104 to resume administration of negative pressure to the tissue site 150, which may result in the removal of much of the administered saline solution. The controller 110 may then direct that a first therapeutic compound, such as a Prontosan^{®} solution, be delivered from the first treatment source 160 to the tissue site 150. A solution, such as Prontosan solution, may be administered for controlling possible infection or levels of microbes at the tissue site 150. Other types of antimicrobial solutions or other forms of therapeutic substances may also be delivered to the tissue site 150. Once again, the negative-pressure source 104 may be powered down for an appropriate period of time, as determined by the controller 110, to allow the first therapeutic compound to take effect at the tissue site 150. Following the appropriate time period, the negative-pressure source 104 may resume operation and may deliver negative pressure to the tissue site 150, thus removing at least a portion of the first therapeutic compound from the tissue site 150. The controller 110 may also use feedback from the diagnostic module 140 to vary the level of negative pressure supplied by the negative-pressure source 104 to the tissue site 150. It should be noted that the controller 110 may automatically adjust the amount of time either form of treatment fluid remains at the tissue site 150, which may at least partially be due to feedback from the diagnostic module 140. Depending on the condition of the tissue site 150, which may in part be determined based on data received from the diagnostic module 140, the controller 110 may direct that additional cycles of alternating between two or more forms of fluid instillation therapy are warranted.

For example, as previously mentioned, one parameter measured by a sensor of the sensor device 142 of the diagnostic module 140 may be pH. Raised pH of tissue sites, such as wounds, has been shown to be connected with several factors of wound conditions such as slower healing rates, elevated protease activity, and higher oxygen (O2) concentrations. In combination with measurement of other parameters, such as temperature and oxygenation of the tissue site, pH measurements may be used to determine that it would be beneficial to instill a solution that would lower the pH at the tissue site. For example, a pre-prepared solution at a preferred pH, such as 0.9% saline which has a typical pH of 5.5-6, may be included in the solution source 114 of the therapy system 100, for instillation to the tissue site. Solutions that are pH-controlled may also be included in the solution source 114, such as for example Phosphate-buffered Saline (PBS), Dulbecco's Phosphate-buffered Saline (DPBS), Hank's Balanced Salt Solution (HBSS), and Earle's Balanced Salt Solution (EBSS). Alternatively or additionally, a means to dose a standard saline solution to alter its pH may be provided by including a dosing solution in either of the first treatment source 160 or second treatment source 162 for addition to a standard saline solution in the solution source 114. Including a dosing solution, for example in the first treatment source 160, for adjusting the pH of a standard saline solution of the solution source 114 may allow for a solution with variable pH levels to be delivered to the tissue site 150 based on the particular condition of the tissue site 150. In such embodiments, the second treatment source 162 may contain an additional wound healing compound which may be added to the instillation fluid from the solution source 114 based on the interpretation by the controller 110 of diagnostic information from the one or more sensors of the diagnostic module 140. For example, either the first treatment source 160 or the second treatment source 162 may include an antimicrobial agent that may be added to the instillation fluid based on a determination by the controller 110 that an infection exists at the tissue site 150.

As previously mentioned, the sensor device 142 of the diagnostic module 140 may include a temperature sensor, which may be used to track the temperature of the tissue site 150 at one or more locations over time. For example, should the controller 110 receive data from the diagnostic module 140 indicating that the temperature of the tissue site 150 has been increasing for an amount of time, the controller 110 may make the determination that an infection is present at the tissue site 150. In response, the controller 110 may direct that particular treatment compound, such as one from either or both of the first treatment source 160 and second treatment source 162, be administered to the tissue site 150.

In additional or alternative embodiments, the diagnostic module 140 may be configured to monitor the administration of particular therapeutic compounds, such as honey, to the tissue site 150. In such embodiments, the sensor device 142 of the diagnostic module 140 may include a glucose sensor. For example, a form of medical grade honey may be administered from the first treatment source 160 to the tissue site 150. A glucose sensor included as part of the sensor device 142 may be configured to monitor glucose levels at the tissue site 150, and the controller 110 may use such measurements to determine whether levels of glucose fall below a pre-determined threshold or out of a therapeutic range. Based on this feedback from the diagnostic module 140, the controller 110 may then direct that additional honey from the first treatment source 160 be delivered to the tissue site 150.

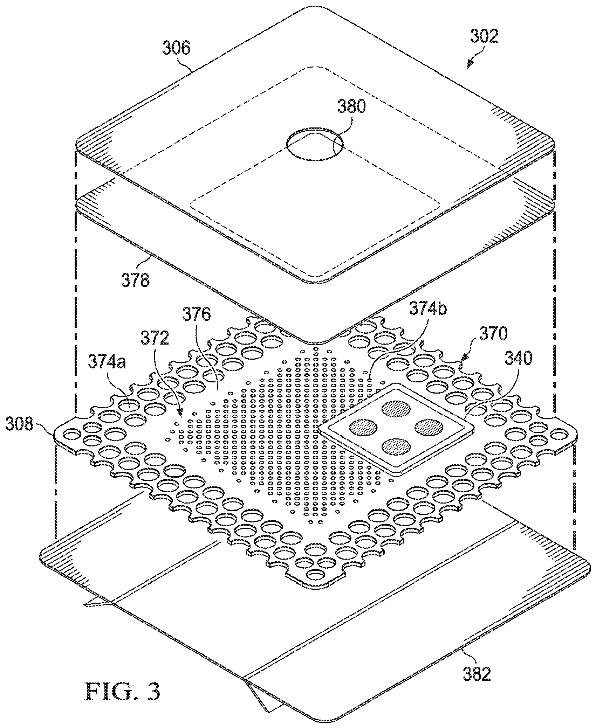

Figure 3 is an exploded view of an example embodiment of a dressing 302 for use as part of the therapy system 100, showing some further detail and additional features. Dressing 302 may include a tissue interface 308 and a cover 306. A diagnostic module 340 may also be included as part of the dressing 302, and may be positioned between the tissue interface 308 and the cover 306. Further, in some embodiments, the dressing 302 may include additional components or layers, such as for example, an absorbent layer and/or one or more manifold layers.

In some embodiments, the tissue interface 308 may have a periphery 370 surrounding a central portion 372, and a plurality of apertures 374 disposed throughout the periphery 370 and the central portion 372. The tissue interface 308 may also have a border 376 substantially surrounding the central portion 372 and positioned between the central portion 372 and the periphery 370. The border 376 may be free of the apertures 374. The tissue interface 308 may be adapted to cover the tissue site as well as the tissue surrounding the tissue site, such that the central portion 372 of the tissue interface 308 is positioned adjacent to or proximate to the tissue site, and the periphery 370 is positioned adjacent to or proximate to tissue surrounding the tissue site. Further, the apertures 374 in the tissue interface 308 may be in fluid communication with the tissue site and tissue surrounding the tissue site. In some embodiments, the dressing 302 may further include an additional structure for placement against or within the tissue site, such as a wound filler.

The apertures 374 in the tissue interface 308 may have any shape, such as for example, circles, squares, stars, ovals, polygons, slits, complex curves, rectilinear shapes, triangles, or other shapes. As shown in Figure 3, each of the plurality of apertures 374 may be substantially circular in shape. Each of the plurality of apertures 374 may have an area, which may refer to an open space or open area defining each of the plurality of apertures 374. In some embodiments, the area of the apertures 374 in the periphery 370 may be larger than the area of the apertures 374 in the central portion 372 of the tissue interface 308. The size and configuration of the plurality of apertures 374 may be designed to control the adherence of the cover 306 to an epidermis surrounding a tissue site.

In some embodiments, the plurality of apertures 374 positioned in the periphery 370 of the tissue interface 308 may be apertures 374a. Additionally, the plurality of apertures 374 positioned in the central portion 372 of the tissue interface 308 may be apertures 374b. Each of the apertures 374a and 374b may vary in size. However, in some embodiments, the apertures 374a may have a diameter between about 9 millimeters to about 11 millimeters. The apertures 374b may have a diameter between about 1.5 millimeters to about 3 millimeters. Furthermore, the spacing between each of the apertures 374a and 374b may also vary depending on the specific embodiment. For example, in some embodiments, the diameter of each of the apertures 374a may be separated from one another by a distance of between about 2.5 millimeters to about 3.5 millimeters. Further, a center of one of the apertures 374b may be separated from a center of another of the apertures 374b in a first direction by a distance of between about 2.5 millimeters to about 3.5 millimeters. In a second direction transverse to the first direction, the center of one of the apertures 374b may be separated from the center of another of the apertures 374b by a distance of between about 2.5 millimeters to about 3.5 millimeters. As shown in Figure 3, the distances may be increased for the apertures 374b in the central portion 372 being positioned proximate to or at the border 376 as compared to the apertures 374b positioned away from the border 376. Importantly, the dimensions of each section of the tissue interface 308, such as the periphery 370, the center portion 372, and the border 376 may vary based on the particular application of the dressing 302.

The tissue interface 308 may be a soft, pliable material suitable for providing a fluid seal with a tissue site. For example, the tissue interface 308 may comprise a silicone gel, a soft silicone, hydrocolloid, hydrogel, polyurethane gel, polyolefin gel, hydrogenated styrenic copolymer gel, a foamed gel, a soft closed-cell foam such as polyurethanes and polyolefins coated with an adhesive, polyurethane, polyolefin, or hydrogenated styrenic copolymers. The tissue interface 308 may have a thickness between about 500 micrometers and about 1,000 micrometers. Further, in some embodiments, the tissue interface 308 may be comprised of hydrophobic or hydrophilic materials.

In some embodiments (not shown), the tissue interface 308 may be a hydrophobic-coated material. For example, the tissue interface 308 may be formed by coating a spaced material, such as, for example, woven, nonwoven, molded, or extruded mesh with a hydrophobic material. The hydrophobic material for the coating may be a soft silicone, for example.

The adhesive 378 may be in fluid communication with the plurality of apertures 374 in at least the periphery 370 of the tissue interface 308. In this manner, the adhesive 378 may be in fluid communication with tissue surrounding a tissue site through the plurality of apertures 374 in the tissue interface 308. The adhesive 378 may extend or be passed through the plurality of apertures 374 to contact epidermis for securing the cover 306 to, for example, tissue surrounding a tissue site. The plurality of apertures 374 may provide sufficient contact of the adhesive 378 to the epidermis to secure the cover 306 about a tissue site. The plurality of apertures 374 and the adhesive 378 may also be configured to permit release and repositioning of the cover 306 about a tissue site.

In some embodiments, an additional or alternative attachment device may be used to secure the cover 306 about the tissue site. For example, double-sided tape, paste, hydrocolloid, hydrogel, silicone gel, or organogel may be used. Furthermore, thicker adhesives, or combinations of adhesives, may be applied in some embodiments to improve seals and to reduce leaks. Additionally, any of the plurality of the apertures 374 may be adjusted in size and number to maximize the surface area of the adhesive 378 in fluid communication through the plurality of the apertures 374 for a particular application or geometry of the tissue interface 308.

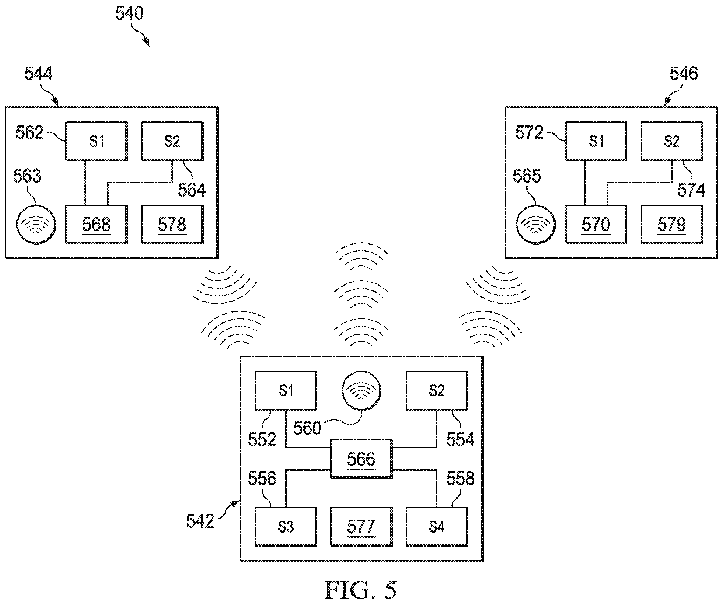

The adhesive 378 may be a medically-acceptable adhesive. The adhesive 378 may also be flowable. For example, the adhesive 378 may comprise an acrylic adhesive, rubber adhesive, high-tack silicone adhesive, polyurethane, or other adhesive substance. In some embodiments, the adhesive 378 may be a pressure-sensitive adhesive, such as an acrylic adhesive with coating weight of 15 grams/m² (gsm) to 70 grams/m2 (gsm). The adhesive 378 may be a layer having substantially the same shape as the periphery 370 of the tissue interface 308, and thus have a large central aperture, as shown in Figure 5. In some embodiments, the layer of the adhesive 378 may be continuous or discontinuous. Discontinuities in the adhesive 378 may be provided by apertures (not shown) in the adhesive 378. Apertures in the adhesive 378 may be formed after application of the adhesive 378 or by coating the adhesive 378 in patterns on a carrier layer, such as, for example, a side of the cover 306 adapted to face the epidermis. Further, apertures in the adhesive 378 may be sized to control the amount of the adhesive 378 extending through the plurality of the apertures 374 in the tissue interface 308 to reach the epidermis. Apertures in the adhesive 378 may also be sized to enhance the Moisture Vapor Transfer Rate (MVTR) of the cover 306, described in further detail below.

Factors that may be utilized to control the adhesion strength of the cover 306 may include the diameter and number of the plurality of the apertures 374 in the tissue interface 308, the thickness of the tissue interface 308, the thickness and amount of the adhesive 378, and the tackiness of the adhesive 378. An increase in the amount of the adhesive 378 extending through the plurality of the apertures 374 may correspond to an increase in the adhesion strength of the cover 306. A decrease in the thickness of the tissue interface 308 may correspond to an increase in the amount of adhesive 378 extending through the plurality of the apertures 374. Thus, the diameter and configuration of the plurality of the apertures 374, the thickness of the tissue interface 308, and the amount and tackiness of the adhesive 378 utilized may be varied to provide a desired adhesion strength for the cover 306. For example, in some embodiments, the thickness of the tissue interface 308 may be about 200 micrometers, the adhesive 378 may be a layer having a thickness of about 30 micrometers and a tackiness of 2000 grams per 25 centimeter wide strip, and the diameter of the apertures 374a in the tissue interface 514 may be about 10 millimeters.

Still referring primarily to Figure 3, a release liner 382 may be attached to or positioned adjacent to the tissue interface 308 to protect the adhesive 378 prior to application of the dressing 302 to the tissue site. Prior to application of the dressing 302 to the tissue site, the tissue interface 308 may be positioned between the cover 306 and the release liner 382. Removal of the release liner 382 may expose the tissue interface 308 and the adhesive 378 for application of the dressing 302 to the tissue site. The release liner 382 may also provide stiffness to assist with, for example, deployment of the dressing 302. The release liner 382 may be, for example, a casting paper, a film, or polyethylene. Further, the release liner 382 may be a polyester material such as polyethylene terephthalate (PET), or similar polar semicrystalline polymer. A release agent may be disposed on a side of the release liner 382 that is configured to contact the tissue interface 308. For example, the release agent may be a silicone coating and may have a release factor suitable to facilitate removal of the release liner 382 by hand and without damaging or deforming the dressing 302. In some embodiments, the release agent may be fluorosilicone. In other embodiments, the release liner 382 may be uncoated or otherwise used without a release agent.

The peripheral portions of the cover 306 may be positioned proximate to the periphery 370 of the tissue interface 308 such that a central portion of the cover 306 and the central portion 372 of the tissue interface 308 define an enclosure. The adhesive 378 may be positioned at least between the peripheral portions of the cover 306 and the periphery 370 of the tissue interface 308. The cover 306 may cover the tissue site and the tissue interface 308 to provide a fluid seal and a sealed space between the tissue site and the cover 306. Further, the cover 306 may cover other tissue, such as a portion of epidermis, surrounding the tissue site to provide the fluid seal between the cover 306 and the tissue site. In some embodiments, a portion of the peripheral portion of the cover 306 may extend beyond the periphery 370 and into direct contact with tissue surrounding the tissue site. In some embodiments, the peripheral portion of the cover 306, for example, may be positioned in contact with tissue surrounding the tissue site to provide the sealed space without the tissue interface 308. Thus, the adhesive 378 may also be positioned at least between the peripheral portion of the cover 306 and tissue, such as the epidermis, surrounding the tissue site. The adhesive 378 may be disposed on a surface of the cover 306 adapted to face the tissue site and the tissue interface 308. Additionally, the cover 306 may include an aperture 380, which in some embodiments may be generally positioned in a central portion of the cover 306. The aperture 380 may allow for fluid communication between a sealed space provided by the cover 306 and including a tissue site, and one or more conduits for conducting negative pressure and/or for delivering therapeutic fluids to the tissue site.

The cover 306 may be formed from any material that allows for a fluid seal, such as any of the materials of the cover 106. The cover 306 may be vapor permeable and liquid impermeable, thereby allowing vapor and inhibiting liquids from exiting the sealed space provided by the cover 306. In other embodiments, a low or no vapor transfer drape might be used. The cover 306 may comprise a range of medically suitable films having a thickness between about 15 microns (µm) to about 50 microns (µm). The tissue interface 308 may be adapted to transfer fluid away from a tissue site rather than store the fluid. For example, as fluid, such as wound exudate, is drawn away from a tissue site, the fluid may pass through the tissue interface 308 in response to the application of negative pressure to the dressing 302, and travel upwards towards the cover 306. Once the fluid reaches the cover 306, the fluid may be drawn through the aperture 380 in the cover 306, out of the dressing 302, and into a fluid conductor, such as the conduit 128 of Figure 1.

The diagnostic module 340 may be positioned within the dressing 302, more specifically between the tissue interface 308 and the cover 306. The diagnostic module 340 may be positioned so that as the tissue interface 308 comes into contact with fluid from a tissue site, the fluid may pass through the apertures 374 of the tissue interface 308 and come into contact with the diagnostic module 340. As the fluid from the tissue site comes into contact with the one or more sensors of the diagnostic module 340, the sensors may detect and/or measure one or more parameters related to the tissue site, such as for example, pH, temperature, humidity, as well as others.

Figure 4 is a schematic diagram of an example embodiment of a portion of the therapy system 100 of Figure 1, showing some further detail and additional features. For example, in Figure 4, features of an example embodiment of dressing 402 are shown in conjunction with an example embodiment of a diagnostic module 440. As shown, the dressing 402 may include a tissue interface 408 positioned on or disposed within tissue site 150 as well as a cover 406. In the example embodiment shown in Figure 4, the diagnostic module 440 may include multiple sensing devices, which may be employed at various locations within the dressing 402 and may be suitable for providing relevant feedback to other components of the therapy system 100. For example, the diagnostic module 440 may include a central sensor device 442, as well as multiple remote sensor devices, such as first remote sensor device 444 and second remote sensor device 446. As shown in Figure 4, the central sensor device 442 may be positioned on the tissue interface 408, while the first remote sensor device 444 and the second remote sensor device 446 may be positioned at other locations, which may also be under the cover 406. For example, the first remote sensor device 444 may be positioned on a portion of the epidermis 445 immediately adjacent to the tissue site 150, such as at a periwound region, while the second remote sensor device 446 may be positioned on the epidermis 445 at a greater distance away from the tissue site 150. Additionally or alternatively, one or more of the sensing devices included as part of the diagnostic module 440 may be placed on or positioned within one or more other components of the therapy system 100.

Figure 5 is a schematic diagram of a diagnostic module 540 for use with the therapy system 100 of Figure 1, illustrating some additional details related to some embodiments. For example, similar to the diagnostic module 440 of Figure 4, the diagnostic module 540 of Figure 5 may include multiple sensing devices, which may be employed at various locations within the therapy system 100, such as within the dressing 102, the dressing interface 107, or other component of the therapy system 100. The diagnostic module 540 may include a central sensor device 542, a first remote sensor device 544, and a second remote sensor device 546. Each of the sensing devices may include one or more sensors. In some embodiments, the individual sensors of the multiple sensing devices may measure the same parameter at different locations, such as within the tissue site 150 and in a periwound region, while in additional or alternative embodiments, the individual sensors may be for measuring different parameters. For example, the central sensor device 542 may include a first sensor 552, a second sensor 554, a third sensor 556, and a fourth sensor 558. Additionally, the first remote sensor device 544 may include a first sensor 562 and a second sensor 564. Further, the second remote sensor device 546 may also include a first sensor 572 and a second sensor 574. In some embodiments, the first sensor 552 of the central sensor device 542, the first sensor 562 of the first remote sensor device 544, and the first sensor 572 of the second remote sensor device 546 may be selected or configured to detect and/or measure a first parameter, which in some instances may be pH. Thus, by being configured to detect and/or measure the same parameter at different locations within the therapy system 100, the diagnostic module 540 in conjunction with other components of the therapy system 100 may be able to determine if a particular parameter is localized to a specific portion of a tissue site or portion of the therapy system 100, or if the particular parameter, such as pH, oxygenation, contact pressure, water loss, etc., is more systemic across multiple locations in and around a tissue site or within the therapy system 100. Additionally, the diagnostic module 540, in conjunction with other components of the therapy system 100, may be able to compare measurements of a particular parameter taken by each of the first sensor 552 of the central sensor device 542, the first sensor 562 of the first remote sensor device 544, and the first sensor 572 of the second remote sensor device 546.

Similarly, in some embodiments, the second sensor 554 of the central sensor device 542, the second sensor 564 of the first remote sensor device 544, and the second sensor 574 of the second remote sensor device 546 may be selected or configured to detect and/or measure a second parameter. In one example embodiment, each of the first sensors 552, 562, and 572 may be configured to measure pH level, for example the pH level of wound exudates that come into contact with each of the first sensors 552, 562, and 572, while each of the second sensors 554, 564, and 574 may be configured to measure the oxygen concentration within the portion of the dressing 102 or therapy system 100 adjacent to each of the second sensors 554, 564, and 574. Additionally, the third sensor 556 of the central sensor device 542 may be configured to measure a third parameter, for example temperature, in the vicinity of the central sensor device 542, while the fourth sensor 558 may be configured to measure a fourth parameter, for example the glucose level, in wound exudates that come into contact with the central sensor device 542. It should be noted that any or all of the individual sensors discussed with respect to each of the central sensor device 542, the first remote sensor device 544, and second remote sensor device 546 may be configured or interchanged with other sensors to detect and/or measure a different one or more parameters. Furthermore, additional remote sensor devices, which may include additional sensors, such as a fourth remote sensor device (not shown) and a fifth remote sensor device (not shown) may be included to detect and/or measure either the same parameter(s) as the central sensor device or a different one or more parameter(s).

Still referring primarily to Figure 5, the central sensor device 542 may include a communications module 560. The communications module 560 may be configured to exchange data with a communications device 111 that is part of the therapy system 100, such as a communications device that is incorporated within the controller 110 and/or the therapy unit 101 of Figure 2. Additionally, each of the first remote sensor device 544 and the second remote sensor device 546 may also include a communications component, which may be configured for exchanging data with the communications module 560 of the central sensor device 542. For example, the first remote sensor device 544 may include a first communications component 563, and the second remote sensor device 546 may include a second communications component 565. In some embodiments, each of the first communications component 563 and the second communications component 565 may be configured to transmit data collected by the sensors on the first remote sensor device 544 and the second remote sensor device 546, respectively, to the communications module 560 of the central sensor device 542. The first communications component 563, the second communications component 565, and the communications module 560 may be configured to exchange data using the Bluetooth^{®} 4.0 protocol, as well as through other wired or wireless communication protocols.

The central sensor device 542 may also include a processing unit 566 for receiving and processing signals and data from each of the sensors onboard the central sensor device 542. Thus, in the example embodiment shown in Figure 5, the processing unit 566 may be configured to receive and process data from the first sensor 552, the second sensor 554, the third sensor 556, and the fourth sensor 558. Additionally, the processing unit 566 may be configured to exchange data with the remote sensor devices, such as the first remote sensor device 544 and the second remote sensor device 546, via the communications module 560. Each of the remote sensor devices, such as first remote sensor device 544 and second remote sensor device 546 may also include a processor, such as first processor 568 and second processor 570, respectively. For example, the first processor 568 may receive and process data from each of the first sensor 562 and the second sensor 564 of the first remote sensor device 544, and instruct the communications component 563 of the first remote sensor device 544 to transmit the data from the sensors to the communications module 560 of the central sensor device 542. Similarly, the second processor 570 may receive and process data from each of the first sensor 572 and second sensor 574 of the second remote sensor device 546, and instruct the communications component 565 of the second remote sensor device 546 to transmit the data from the sensors to the communications module 560 of the central sensor device 542. In some embodiments, the processing unit 566 of the central sensor device 542 may collect and process the data transmitted from the first remote sensor device 544 and the second remote sensor device 546 in conjunction with data from the sensors onboard the central sensor device 542. The processing unit 566 may then also instruct the communications module 560 to transmit the data from the sensors of one or all of the central sensor device 542, the first remote sensor device 544 and the second remote sensor device 566 to the communications device 111 and/or controller 110.

Still referring primarily to Figure 5, each of the sensor devices, such as the central sensor device 542, the first remote sensor device 544, and the second remote sensor device 546, may include a power supply. Thus, the central sensor device 542 may include central power supply 577, the first remote sensor device 544 may include first power supply 578, and the second remote sensor device 546 may include second power supply 579. In some embodiments, each of the power supplies may be a battery.

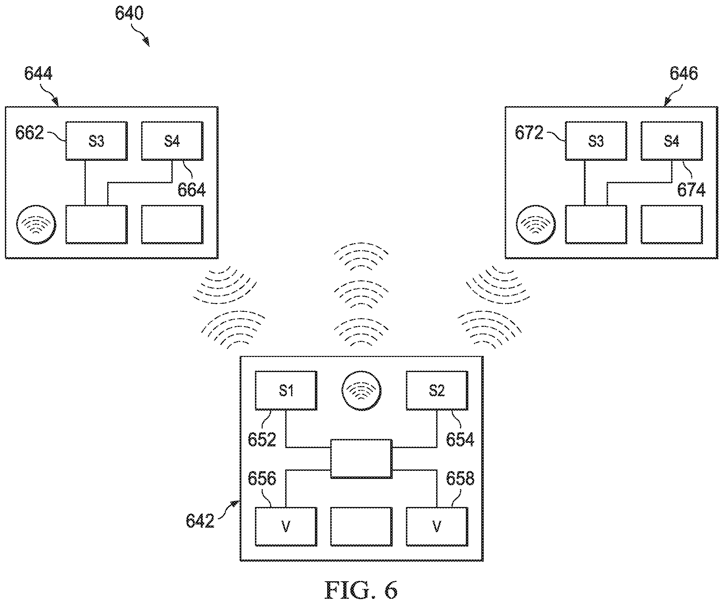

Figure 6 is a schematic diagram of a diagnostic module 640 for use with the therapy system 100 of Figure 1, illustrating some additional details related to additional embodiments. For example, similar to the diagnostic unit 540 of Figure 5, the diagnostic unit 640 of Figure 6 may include multiple sensing devices, which may be employed at various locations within the therapy system 100. The diagnostic unit 640 may include a central sensor device 642, a first remote sensor device 644, and a second remote sensor device 646. Similarly to the sensing devices of the diagnostic unit 540, each of the sensing devices of the diagnostic unit 640 may include one or more sensors. However, in the embodiment illustrated in Figure 6, the sensors of the remote sensing devices, such as the first remote sensor device 644 and the second remote sensor device 646, may be configured to measure different parameters from the sensors of the central sensor device 642. For example, the central sensor device 642 may include a first sensor 652 and a second sensor 654 that are configured to measure a first parameter and a second parameter, respectively. However, unlike the central sensor device 542 of Figure 5, which is shown as also including third and fourth sensors, the central sensor device 642 of Figure 6 does not include a third or fourth sensor. Rather, the central sensor device 642 is shown as having vacant ports or sockets, such as first port 656 and second port 658, where additional sensors, such as a third sensor and fourth sensor, could be connected. Thus, it is also worth noting, that the sensing devices as disclosed herein, may offer the option of customization or switching out individual sensors based on the desired specific parameters to be measured.

As mentioned, the remote sensor devices, such as the first remote sensor device 644 and the second remote sensor device 646, may include individual sensors that measure parameters different from those measured by the sensors of the central sensor device 642. Thus, in some embodiments, the first remote sensor device 644 may include a first sensor 662 and a second sensor 664 that are configured to measure a third parameter and a fourth parameter, respectively. Similarly, the second remote sensor device 646 may include a first sensor 672 and a second sensor 674 that are also configured to measure the third parameter and the fourth parameter, respectively. Meanwhile, the first sensor 652 and the second sensor 654 of the central sensor device 642 may be configured to measure a first parameter and a second parameter, respectively. Also worth noting, in some embodiments, the sensors of the second remote sensor device 646 may be configured to measure parameters different from the sensors of the first remote sensor device 644 and the central sensor device 642. Thus, in some embodiments, the sensors, such as the first sensor 672 and the second sensor 674, of the second remote sensor device 646 may be configured to measure a fifth parameter and a sixth parameter, respectively. The sensor devices of the diagnostic module 640, such as the central sensor device 642, the first remote sensor device 644, and the second remote sensor device 646 may otherwise include analogous features and operate analogously to the sensor devices of the diagnostic module 540.

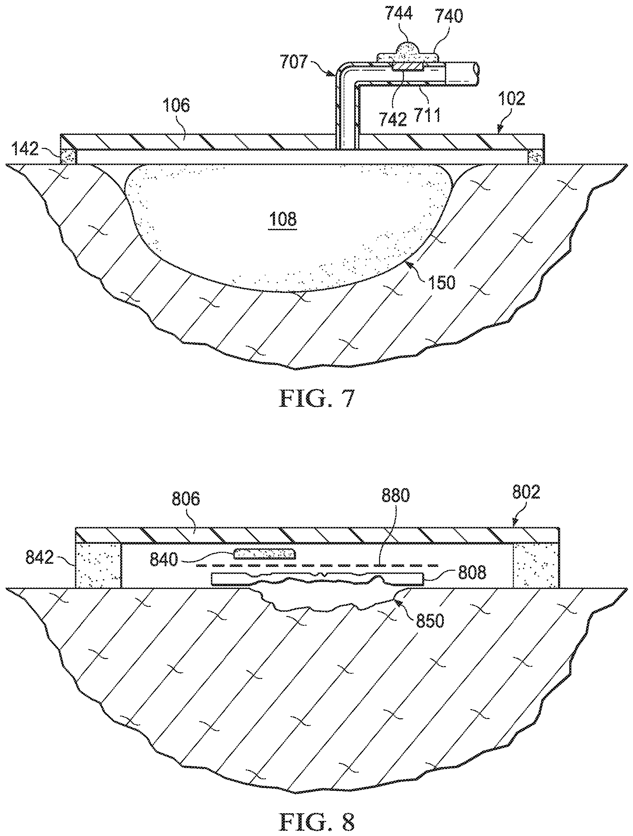

Figure 7 is a schematic diagram of an example embodiment of a portion of the therapy system 100 of Figure 1, showing some additional features. More specifically, in the embodiment illustrated in Figure 7, a diagnostic module, such as diagnostic module 740 may be included as a part of or attached to a dressing interface, such as dressing interface 707. In such embodiments, the diagnostic module 740 may be positioned on or included as part of the dressing interface 707 so that one or more parameters of fluid passing through the dressing interface 707 may be measured. The diagnostic module 740 may include a sensor device 742, which may be configured to measure one or more parameters in the fluid passing through the dressing interface 707. Similarly to other embodiments of sensor devices discussed above, the sensor device 742 may include one or more individual sensors. Thus, as fluids, such as wound exudates, pass from the dressing 102 through the fluid passageway 711 of the dressing interface 707, the fluids may come into contact with the one or more sensors of the sensor device 742, thus allowing the target parameters to be detected and/or measured. The diagnostic module 740 may also include a communications component, such as communications module 744, for transmitting and receiving data to/from one or more other components of the therapy system 100, such as the controller 110. As also previously discussed, the communications module 744 may be of a type configured to wirelessly exchange data, using the Bluetooth^{®} 4.0 protocol, with a communications device 111 that is electrically coupled to the controller 110. The diagnostic module 740 may alternatively or additionally be electrically coupled to the controller 110 via one or more wires.

Figure 8 is a schematic diagram of another example embodiment of a portion of the therapy system 100 of Figure 1, showing additional features. More specifically, Figure 8 illustrates features of an embodiment of the therapy system 100 that may allow for using a diagnostic module to measure a parameter of a dressing material as it is degraded. One or more such measured parameters may allow the therapy system 100 to directly or indirectly determine one or more conditions or properties of a tissue site, such as tissue site 850. For example, the dressing 802 may be configured to cover and provide a sealed space around tissue site 850. The dressing 802 may include a tissue interface 808, which in some instances may be formed of a material designed to degrade in the presence of fluid, such as wound exudates. The dressing 802 may further include a diagnostic module 840, which similar to the diagnostic modules previously discussed, may include one or more sensors for detecting and/or measuring one or more parameters contained within fluids such as wound exudates. Additionally, the dressing 802 may include a material layer, such as membrane 880, for separating the diagnostic unit 840 from the tissue interface 808. For example, the membrane 880 may be a physiologically-neutral perforated membrane, which may allow fluids from the tissue site 850 that may contain traces of degraded tissue interface 808 to pass through the perforations in membrane 880 and to come into contact with the one or more sensors on the diagnostic module 840. The dressing 802 may also include a cover 806 which may provide a sealed space around the other components of the dressing 802 and may be secured to a portion of epidermis surrounding the tissue site 850 with attachment device 842.

Still referring to Figure 8, in some embodiments, the one or more sensors of the diagnostic module 840 may be configured to indirectly measure protease levels in the environment surrounding the tissue site 850. In such embodiments, the sensors of the diagnostic module 840 may be configured to measure the electrical impedance of a doped material, such as the tissue interface 808, as it is degraded, for serving as an indicator of protease activity in the tissue site 850. For example, the tissue interface 808 may be a collagen material, such as the PROMOGRAN PRISMA^{™} Matrix, available from Acelity LP, Inc. of San Antonio, Texas. In such embodiments, the sensor(s) of the diagnostic module 840 may be configured to measure electrical properties of the collagen material of the tissue interface 808. For example, the electrical impedance of the collagen material of the tissue interface 808 may be measured to indirectly determine the level of protease activity in the tissue site 850.

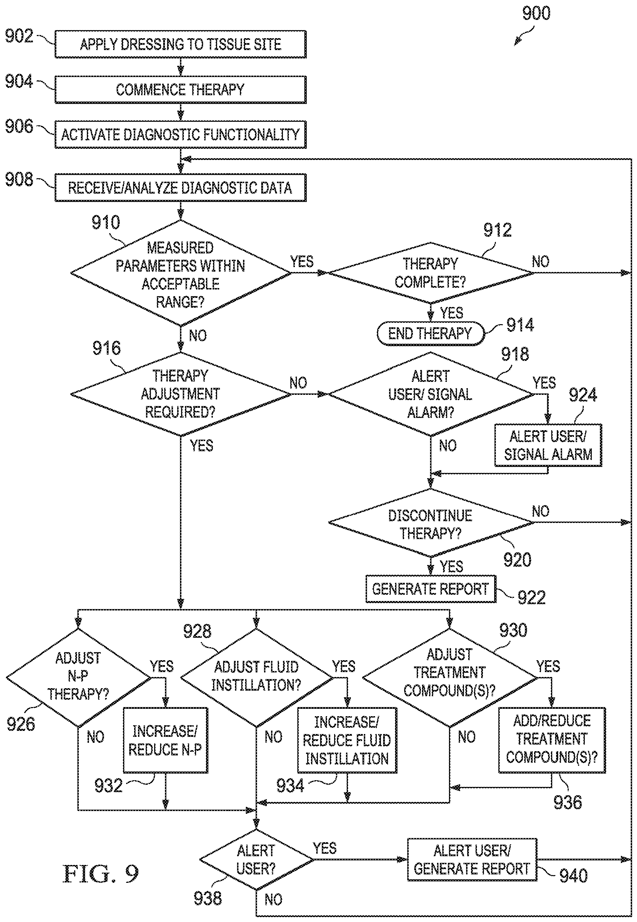

In operation, the therapy system 100 and its various components and features may be used in accordance with the exemplary operating method 900 illustrated in Figure 9. For example, operation of the therapy system 100 may begin with applying a dressing, such as dressing 102, to a tissue site 150, as shown in step 902. The dressing 102 may be fluidly connected to the negative-pressure source 104 by fluid conductor 128 via first dressing interface 107. The therapy system 100 may then be activated to begin delivering therapy, such as negative-pressure therapy, to the dressing 102 and tissue site 150, by fluidly connecting a negative-pressure source, such as negative-pressure source 104, to the dressing 102 and then activating the negative-pressure source 104, as shown in step 904. Step 906 shows the process of activating the diagnostic functionality of the therapy system 100, which may involve initializing a diagnostic module, such as diagnostic module 140, of the therapy system 100. Such initialization of the diagnostic module 140 may include initiating any sensors included as part of the diagnostic module 140, as well as activating a communications module, such as communications module 144, of the diagnostic module 140 to begin exchanging data with a controller, such as controller 110, of the therapy system 100. As part of such an initialization process, the controller 110 may communicate with the diagnostic module 140 to determine the types and specific versions of sensors included in the diagnostic module 140. Once the controller 110 has determined that the individual sensors of the diagnostic module 140 are compatible with the therapy system 100, the diagnostic module 140 may begin receiving signals from the sensors. As the therapy system 100 operates and delivers therapy, such as negative-pressure therapy to the tissue site 150, the one or more sensors of the diagnostic module 140 may become exposed to substances, such as wound exudates, from the tissue site environment, and the sensors may begin to collect data regarding one or more specific parameters. As previously discussed, parameters detected and/or measured by the one or more sensors may include pH of wound exudates, O2 concentration of tissue at the tissue site 150, temperature, humidity within the dressing 102, glucose level in wound exudates, as well as others.

Once the diagnostic module 140 of the therapy system 100 has begun transmitting data from the one or more sensors, the controller 110 of the therapy system 100 may receive and process the data, as depicted in step 908 of method 900. The controller 110 of the therapy system 100 may then determine whether the one or more parameters detected and/or measured by the sensor(s) of the diagnostic module 140 fall within an acceptable range, as shown in step 910. Should the controller 110 determine that the one or more measured parameters are within an acceptable range, the controller 110 may determine, as depicted in step 912, whether the desired therapy for the tissue site 150 has been completed. In some embodiments, if the controller 110 determines that therapy has been completed, the delivery of therapy, such as negative-pressure therapy, may be ceased, as depicted in step 914. However, should the controller 110 determine in step 912 that therapy has not been completed, the therapy system 100 will continue to operate to deliver therapy, and the controller 110 may continue to receive and process data from the diagnostic unit 140, according to steps 908 and 910.

Returning to step 910 of method 900, should it be determined by the controller 110 that the one or more measured parameters are not within an acceptable range, the controller 110 may then determine whether an adjustment to the provided therapy or whether an additional form of therapy is necessary or would be beneficial, as shown in step 916. If it is determined that no adjustment of therapy or additional form(s) of therapy is necessary, the controller 110 may then determine whether an alert should be generated for a user of the therapy system 100 to indicate that one or more parameters are outside of an acceptable range, as shown in step 918. Depending on the particular parameter that falls outside of a prescribed range, the therapy system 100 may also be configured to determine whether a visual or audible alarm should be generated. Such a user alert and/or alarm may then be generated, as illustrated in step 924. Regardless of whether an alert or alarm is generated, the controller 110 may then determine whether therapy should be discontinued given that one or more measured parameters is out of an acceptable range, as shown in step 920. If therapy is to be discontinued, the therapy system 100 may cease to provide therapy, and in some instances, a report indicating the status of the measured parameters along with other operational data may be generated, as illustrated in step 922. Otherwise, the therapy system 100 may continue to provide therapy, and the controller 110 may return to step 908 of method 900.

Referring back to step 916, should the controller 110 determine that an adjustment to therapy is warranted, the controller 110 may then proceed to a series of decision points to determine which type or types of therapy adjustments are needed. For example, as shown in step 926, the controller 110 may assess whether the amount of negative-pressure therapy should be adjusted, and if it is determined that an adjustment is needed, may make such an adjustment as shown in step 932. Additionally, the controller 110 may determine whether an adjustment to fluid instillation therapy is warranted, as depicted in step 928, which in some embodiments may result in the amount of instillation fluid, such as saline solution, to be increased, reduced, or stopped, as depicted in step 934. Further, the controller 110 may also determine whether one or more additional treatment compounds should be administered, as shown in step 930. For example, the controller 110 may determine that an antimicrobial compound should be delivered to the tissue site 150, in which case, as part of step 936, such a compound may be added to the instillation fluid being administered to the tissue site 150.

Regardless of the outcome of the decision points illustrated in steps 926, 928, and 930, the controller 110 may then determine, as depicted in step 938, whether one or more user alerts should be generated. Such an alert may then be subsequently generated and/or a report generated for a user, as shown in step 940. Regardless of such an alert, the therapy system 100 may then continue to operate according to the adjustments needed and determined by the various steps of the method 900, with the controller returning to step 908 to continue to receive and analyze diagnostic data and repeating the subsequent steps in method 900 until it is determined that therapy is completed at one of the appropriate decision points.

The systems, apparatuses, and methods described herein may provide significant advantages. For example, currently, it may be challenging for clinicians to recognize conditions, such as infection, at a tissue site undergoing negative-pressure therapy or fluid instillation therapy. As a result, appropriate adjustments to therapy or administration of additional therapeutic compounds, such as antimicrobial solutions to treat or prevent infection, may not always take place as quickly as desired. Therefore, by providing a therapy system that incorporates real-time diagnostic feedback with respect to parameters at a tissue site, clinicians may be better alerted to developing or changing conditions at the tissue site. Additionally, the therapy systems disclosed herein may also provide output recommendations to users, such as clinicians, for adding, adjusting, or suspending one or more forms of therapy based on the diagnostic feedback.

Furthermore, while many negative-pressure wound therapy systems currently will provide a set therapy based on settings made by a clinician, typically no adjustments to the therapy are made until the system is attended to by a caregiver or other operator. Thus, adjustments are often not made until later time points when a tissue site, such as a wound, can be assessed and alterations to therapy made, if required. As an improvement or solution to this outstanding need, the systems and devices of the present disclosure may offer the ability to incorporate real-time diagnostic feedback into a therapy system, and may further enable improved and automated decision-making for administering therapy to a tissue site. Thus, a fully-automated therapy system with functions such as automated control of negative-pressure therapy and fluid instillation therapy, as well as delivery of one or more therapeutic compounds, may be provided. By including the capability of gathering real-time feedback data from a diagnostic module worn in proximity to the tissue site, therapy adjustments may be quickly made by the therapy system, in some cases almost instantaneously. Operators of therapy systems with such diagnostic capabilities may also be provided with improved information regarding the condition of the tissue site, and therefore may be empowered to more quickly make better decisions regarding therapy applied to the tissue site.

Also worth noting is that the designs and solutions disclosed herein may be sealable so that the therapy system 100 may be able to detect variables associated with multiple tissue sites and/or dressings, and simultaneously administer one or more appropriate forms of therapy to those respective tissue sites. For Example, a single therapy unit 101, such as a V.A.C.ULTA^{™} Unit, may receive feedback from a diagnostic module 140 having remote sensors positioned at multiple tissue sites, and may administer the same or different forms of negative-pressure and/or fluid instillation therapy to the tissue sites based on the respective feedback(s) from the different remote sensors.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 102, the container 112, or both may be eliminated or separated from other components for manufacture or sale. In other example configurations, the controller 110 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described herein may also be combined or replaced by alternative features serving the same, equivalent, or similar purpose without departing from the scope of the invention defined by the appended claims.

## Claims

1. A system for treating a tissue site, comprising:
a dressing (102, 302, 402, 802) adapted to be placed on the tissue site (150);
a diagnostic module (140, 340, 440, 540, 740, 840) adapted to be positioned adjacent the dressing (102, 302, 402, 802) and comprising:
a first sensor (442, 542, 642) configured to detect a pH level of fluid present at the tissue site (150) and to generate a first output based on the detected pH level, the first sensor (442, 542, 642) comprising a first transceiver configured to transmit the first output;
a first remote sensor device (444, 544, 644) comprising a second sensor, wherein the second sensor is configured to detect a secondary pH level and to generate a second output based on the detected secondary pH level, the first remote sensor device (444, 544, 644) comprising a second transceiver configured to transmit the second output generated by the second sensor;
a negative-pressure source (104) adapted to be fluidly coupled to the dressing (102, 302, 402, 802);
a first fluid source adapted to be fluidly coupled to the dressing (102, 302, 402, 802) and to deliver a first fluid to the dressing (102, 302, 402, 802); and
a controller (110) configured to receive the first output and control the negative-pressure source (104) and the fluid source based on the first output from the first sensor and to receive the second output and compare the first output to the second output.

2. The system of claim 1, wherein the first fluid comprises saline.

3. The system of claim 1, wherein the first fluid comprises an antimicrobial solution.

4. The system of claim 1, further comprising:
a second fluid source adapted to be fluidly coupled to the dressing (102, 302, 402, 802) and to deliver a second fluid to the dressing (102, 302, 402, 802);
wherein the controller (110) is further configured to provide an alert to indicate which of the first fluid and second fluid should be delivered to the dressing (102, 302, 402, 802).

5. The system of claim 1, further comprising:
a second fluid source adapted to be fluidly coupled to the dressing (102, 302, 402, 802) and to deliver a second fluid to the dressing (102, 302, 402, 802);
wherein the controller (110) is further configured to control the second fluid source and to regulate delivery of the first fluid and the second fluid based on the first output from the first sensor.

6. The system of claim 1, further comprising a first treatment source (160) configured to provide a first therapeutic compound to the first fluid.

7. The system of claim 1, further comprising:
a first treatment source (160) configured to provide a first therapeutic compound to the first fluid; and
a second treatment source (1620 configured to provide a second therapeutic compound to the first fluid;
wherein the controller (110) is configured to control the first treatment source (160) and the second treatment source (162) based on the first output from the first sensor.

8. The system of claim 1, wherein the controller (110) is configured to receive the first output from the first transceiver via a wireless signal.

9. The system of claim 1, wherein the first remote sensor device is adapted to be positioned proximate the tissue site at a distance separating the first sensor and the first remote sensor device.

## Patentansprüche

1. Ein System zum Behandeln einer Gewebestelle, aufweisend:
einen Verband (102, 302, 402, 802), der angepasst ist, um auf der Gewebestelle (150) platziert zu werden;
ein Diagnosemodul (140, 340, 440, 540, 740, 840), das angepasst ist, um angrenzend an den Verband (102, 302, 402, 802) positioniert zu werden, und aufweisend:
einen ersten Sensor (442, 542, 642), der konfiguriert ist, um einen pH-Wert des Fluids zu erfassen, das an der Gewebestelle (150) vorhanden ist, und einen ersten Ausgang basierend auf dem erfassten pH-Wert zu erzeugen, der erste Sensor (442, 542, 642) aufweisend einen ersten Sendeempfänger, der konfiguriert ist, um den ersten Ausgang zu übertragen;
eine erste entfernte Sensorvorrichtung (444, 544, 644), aufweisend einen zweiten Sensor, wobei der zweite Sensor konfiguriert ist, um einen sekundären pH-Wert zu erfassen und einen zweiten Ausgang basierend auf dem erfassten sekundären pH-Wert zu erzeugen, die erste entfernte Sensorvorrichtung (444, 544, 644) aufweisend einen zweiten Sendeempfänger, der konfiguriert ist, um die zweite Ausgabe zu übertragen, die durch den zweiten Sensor erzeugt wird;
eine Unterdruckquelle (104), die angepasst ist, um mit dem Verband (102, 302, 402, 802) fluidisch gekoppelt zu sein;
eine erste Fluidquelle, die angepasst ist, um mit dem Verband (102, 302, 402, 802) fluidisch gekoppelt zu werden, und um ein erstes Fluid an den Verband (102, 302, 402, 802) abzugeben; und
eine Steuerung (110), die konfiguriert ist, um den ersten Ausgang zu empfangen und die Unterdruckquelle (104) und die Fluidquelle basierend auf der ersten Ausgabe von dem ersten Sensor zu steuern und den zweiten Ausgang zu empfangen und den ersten Ausgang mit dem zweiten Ausgang zu vergleichen.

2. Das System nach Anspruch 1, wobei das erste Fluid eine Kochsalzlösung aufweist.

3. Das System nach Anspruch 1, wobei das erste Fluid eine antimikrobielle Lösung aufweist.

4. Das System nach Anspruch 1, ferner aufweisend:
eine zweite Fluidquelle, die angepasst ist, um mit dem Verband (102, 302, 402, 802) fluidisch gekoppelt zu werden, und um ein zweites Fluid an den Verband (102, 302, 402, 802) abzugeben;
wobei die Steuerung (110) ferner konfiguriert ist, um eine Warnung bereitzustellen, um anzuzeigen, welche von dem ersten Fluid und dem zweiten Fluid an den Verband (102, 302, 402, 802) abgegeben werden sollte.

5. Das System nach Anspruch 1, ferner aufweisend:
eine zweite Fluidquelle, die angepasst ist, um mit dem Verband (102, 302, 402, 802) fluidisch gekoppelt zu werden, und um ein zweites Fluid an den Verband (102, 302, 402, 802) abzugeben;
wobei die Steuerung (110) ferner konfiguriert ist, um die zweite Fluidquelle zu steuern und eine Abgabe des ersten Fluids und des zweiten Fluids basierend auf der ersten Ausgabe von dem ersten Sensor zu regulieren.

6. Das System nach Anspruch 1, ferner aufweisend eine erste Behandlungsquelle (160), die konfiguriert ist, um eine erste therapeutische Verbindung an das erste Fluid bereitzustellen.

7. Das System nach Anspruch 1, ferner aufweisend:
eine erste Behandlungsquelle (160), die konfiguriert ist, um eine erste therapeutische Verbindung an das erste Fluid bereitzustellen; und
eine zweite Behandlungsquelle (1620, die konfiguriert ist, um eine zweite therapeutische Verbindung an das erste Fluid bereitzustellen;
wobei die Steuerung (110) konfiguriert ist, um die erste Behandlungsquelle (160) und die zweite Behandlungsquelle (162) basierend auf der ersten Ausgabe von dem ersten Sensor zu steuern.

8. Das System nach Anspruch 1, wobei die Steuerung (110) konfiguriert ist, um den ersten Ausgang von dem ersten Sendeempfänger über ein drahtloses Signal zu empfangen.

9. Das System nach Anspruch 1, wobei die erste entfernte Sensorvorrichtung angepasst ist, um in der Nähe der Gewebestelle in einem Abstand positioniert zu werden, der den ersten Sensor und die erste entfernte Sensorvorrichtung trennt.

## Revendications

1. Système pour traiter un site tissulaire, comprenant :
un pansement (102, 302, 402, 802) conçu pour être placé sur le site tissulaire (150) ;
un module de diagnostic (140, 340, 440, 540, 740, 840) conçu pour être positionné adjacent au pansement (102, 302, 402, 802) et comprenant :
un premier capteur (442, 542, 642) configuré pour détecter un niveau de pH d'un fluide présent au niveau du site tissulaire (150) et pour générer une première sortie sur la base du niveau de pH détecté, le premier capteur (442, 542, 642) comprenant un premier émetteur-récepteur configuré pour émettre la première sortie ;
un premier dispositif capteur distant (444, 544, 644) comprenant un second capteur, dans lequel le second capteur est configuré pour détecter un niveau de pH secondaire et pour générer une seconde sortie sur la base du niveau de pH secondaire détecté, le premier dispositif capteur distant (444, 544, 644) comprenant un second émetteur-récepteur configuré pour émettre la seconde sortie générée par le second capteur ;
une source de pression négative (104) conçue pour être accouplée de manière fluidique au pansement (102, 302, 402, 802) ;
une première source de fluide conçue pour être accouplée fluidiquement au pansement (102, 302, 402, 802) et pour administrer un premier fluide au pansement (102, 302, 402, 802) ; et
un dispositif de commande (110) configuré pour recevoir la première sortie et commander la source de pression négative (104) et la source de fluide sur la base de la première sortie provenant du premier capteur et pour recevoir la seconde sortie et comparer la première sortie à la seconde sortie.

2. Système selon la revendication 1, dans lequel le premier fluide comprend une solution saline.

3. Système selon la revendication 1, dans lequel le premier fluide comprend une solution antimicrobienne.

4. Système selon la revendication 1, comprenant en outre :
une seconde source de fluide conçue pour être accouplée fluidiquement au pansement (102, 302, 402, 802) et pour administrer un second fluide au pansement (102, 302, 402, 802) ;
dans lequel le dispositif de commande (110) est en outre configuré pour fournir une alerte pour indiquer lequel du premier fluide et du second fluide doit être administré au pansement (102, 302, 402, 802).

5. Système selon la revendication 1, comprenant en outre :
une seconde source de fluide conçue pour être accouplée fluidiquement au pansement (102, 302, 402, 802) et pour administrer un second fluide au pansement (102, 302, 402, 802) ;
dans lequel le dispositif de commande (110) est en outre configuré pour commander la seconde source de fluide et pour réguler l'administration du premier fluide et du second fluide sur la base de la première sortie provenant du premier capteur.

6. Système selon la revendication 1, comprenant en outre une première source de traitement (160) configurée pour fournir un premier composé thérapeutique au premier fluide.

7. Système selon la revendication 1, comprenant en outre :
une première source de traitement (160) configurée pour fournir un premier composé thérapeutique au premier fluide ; et
une seconde source de traitement (1620 configurée pour fournir un second composé thérapeutique au premier fluide ;
dans lequel le dispositif de commande (110) est configuré pour commander la première source de traitement (160) et la seconde source de traitement (162) sur la base de la première sortie provenant du premier capteur.

8. Système selon la revendication 1, dans lequel le dispositif de commande (110) est configuré pour recevoir la première sortie provenant du premier émetteur-récepteur par le biais d'un signal sans fil.

9. Système selon la revendication 1, dans lequel le premier dispositif capteur distant est conçu pour être positionné à proximité du site tissulaire à une distance séparant le premier capteur et le premier dispositif capteur distant.
